# EUROPEAN PATENT SPECIFICATION

(11) **EP 4 188 459 B1**
(45) Date of publication and mention of the grant of the patent: **03.01.2024**
(21) Application number: 21762103.6
(22) Date of filing: 30.07.2021
(51) Int. Cl.: A61L 2/24, A61L 2/10

(54) **CABINET FOR SANITIZING GARMENTS**
SCHRANK ZUR DESINFEKTION VON KLEIDUNGSSTÜCKEN
ARMOIRE POUR LA DÉSINFECTION DE VÊTEMENTS

(30) Priority: 31.07.2020 IT 202000018805
(43) Date of publication of application: 07.06.2023
(73) Proprietor: EVOCA S.p.A., 24030 Valbrembo (BG) (IT)
(72) Inventor: BRUSADELLI, Ettore, 20121 Milano (IT)
(74) Representative: Studio Torta S.p.A.
(86) International application number: PCT/IB2021/056984
(87) International publication number: WO 2022/024070

(56) References cited:
- WO-A1-2018/142013
- CN-U- 210 177 225
- US-A1- 2015 118 107
- US-A1- 2019 070 325
- US-A1- 2020 011 003

## Description

### Technical Field of the Invention

The present invention relates to a cabinet for sanitizing garments.

In particular, the present invention relates to a cabinet of the type comprising substantially parallel back and front walls; a pair of substantially parallel side walls connected to the back and front walls at respective longitudinal edges; and substantially parallel top and bottom walls.

### State of the Art

Generally, the front wall of the cabinet is movable between an open position and a closed position in which the front wall closes an inner compartment of the cabinet.

The cabinet houses therein a support device for at least one garment, and at least one Ultraviolet (UV) radiation source for sanitizing the garment. US2015118107, WO2018/142013 and CN201177225 describe sanitizing cabinets for garments defined by a housing and UV-lamps as sanitizing means

### Object and Summary of the Invention

The known above-described cabinets for sanitizing garments have some drawbacks mainly deriving from a relatively poor effectiveness of the sanitizing treatment.

The object of the present invention is to provide an improved cabinet for sanitizing garments free from the above-described drawbacks and which is easy and cost-effective to manufacture.

According to the present invention, a cabinet for sanitizing garments is provided, as claimed in the appended claims.

### Brief Description of the Drawings

The present invention will now be described with reference to the accompanying drawings, which illustrate a non-limiting example embodiment thereof, wherein:
Figures 1 and 2 are two schematic perspective views, with parts removed for clarity, of a preferred embodiment of the cabinet of the present invention illustrated in two different operating positions;
Figure 3 is a schematic perspective view, with parts in section and parts removed for clarity, of the cabinet of Figures 1 and 2;
Figure 4 is a schematic plan view, with parts removed for clarity, of the cabinet of Figures 1 and 2;
Figures 5 and 6 are two schematic perspective views, with parts removed for clarity, of a variant of the cabinet of Figures 1 and 2.

### Detailed Description of Preferred Embodiments of the Invention

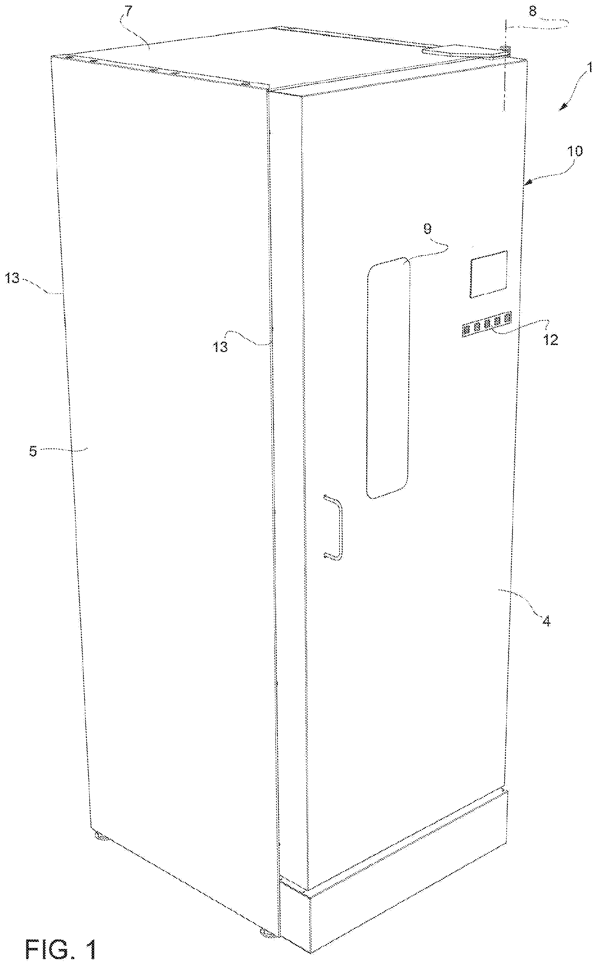

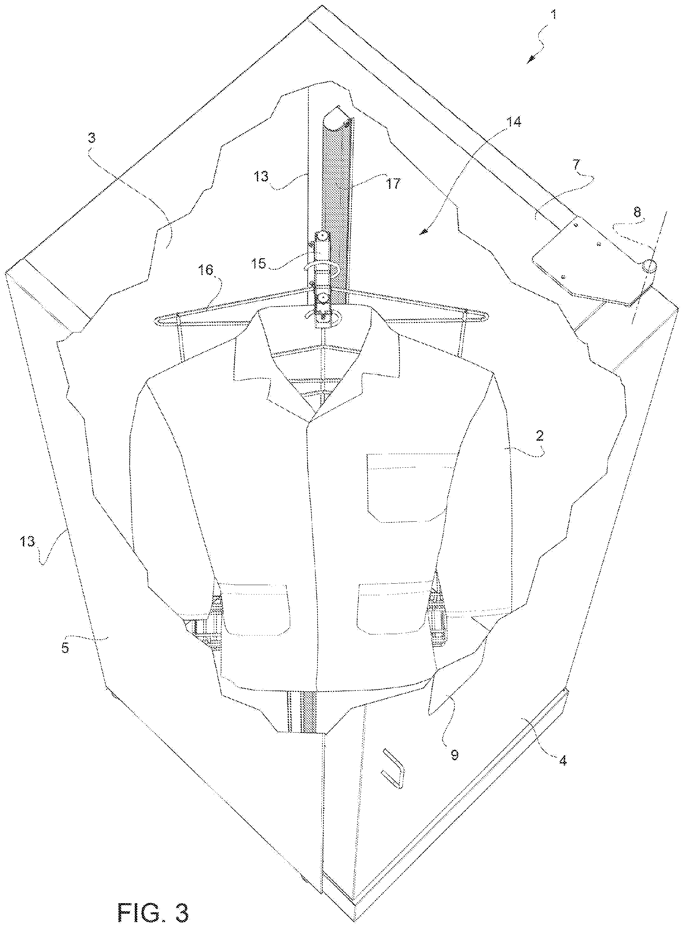

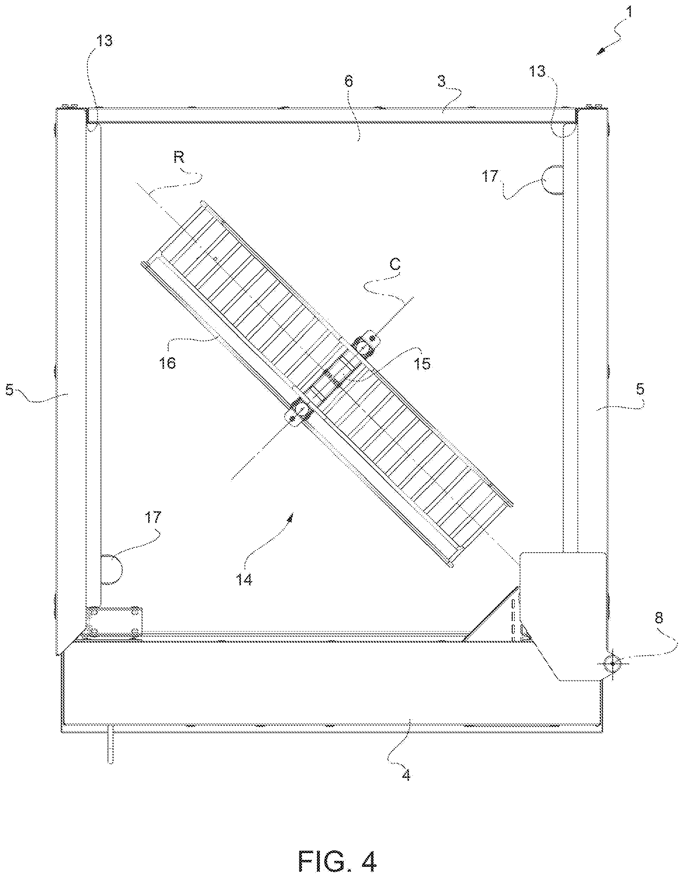

With reference to Figures 1 to 4, a cabinet for sanitizing garments 2 is designated, as a whole, by reference numeral 1.

The cabinet 1 comprises substantially parallel back and front walls 3, 4; a pair of substantially parallel side walls 5 perpendicular to the walls 3 and 4; a bottom wall 6; and a top wall 7 opposite and parallel to the wall 6.

The wall 4 is mounted so as to rotate about a substantially vertical fulcrum axis 8 between an open position and a closed position, is provided with a transparent inspection window 9, and supports an electronic control unit 10.

The unit 10 is provided with a memory 11 for storing a plurality of sanitizing programmes and with a selection keypad 12 for selecting the sanitizing programmes.

Each wall 5 is connected to each of the walls 3, 4 at a longitudinal edge 13 substantially parallel to the axis 8.

The cabinet 1 further comprises a support device 14 comprising a hook 15, which is U-shaped in the example shown, is fixed to the wall 7, and extends in an arrangement plane C substantially perpendicular to a reference plane R passing through two opposite edges 13.

The device 14 further comprises a cloth hook or hanger 16 coupled to the hook 15 so as to maintain at least one garment 2 in a position substantially parallel to the reference plane R.

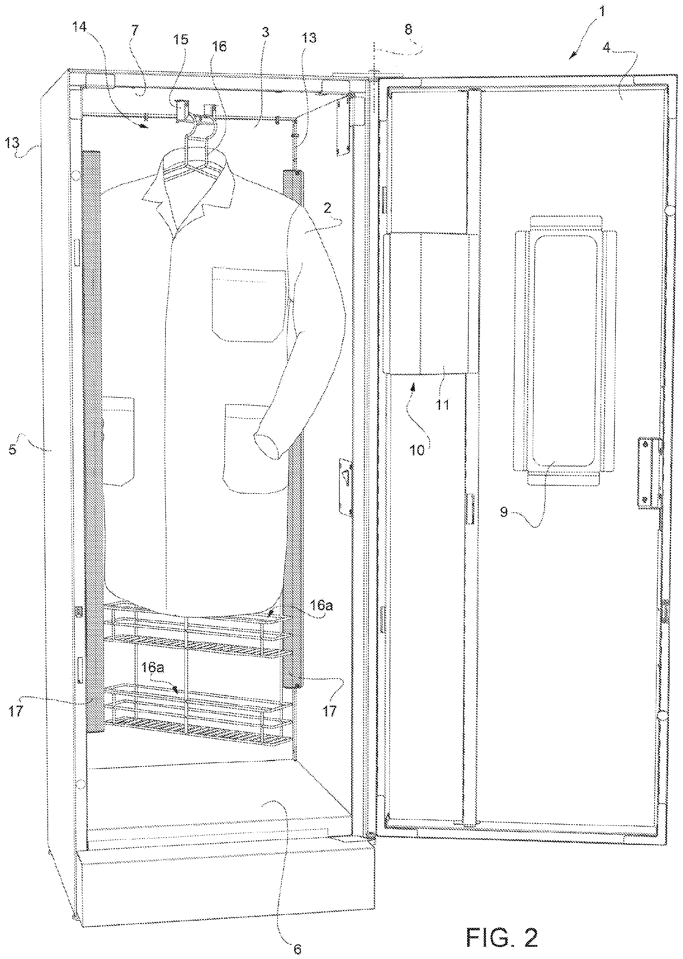

Preferably, as shown in Figure 2, the cloth hanger 16 is an accessory provided not only with a traditional upper shaped crosspiece for hanging up shirts, jackets or overcoats, but also with transverse rods (not visible in Figure 2) for hanging up, for example, pants, and with lower baskets 16a for placing objects or small clothes that one wishes to sanitize.

The cabinet 1 further comprises two Ultraviolet (UV) radiation sources 17, which extend between the walls 6 and 7 parallel to the axis 8, are arranged on opposite sides of the plane R, and are mounted in the proximity of two edges 13 which are arranged on opposite sides of the plane R so as to irradiate the garment 2 with a relatively broad, and thus effective, beam of Ultraviolet (UV) radiations.

In other words, the positioning of the garment 2 in the plane R allows the sources 17 to be mounted at a relatively large distance from the plane R, thus improving effectiveness of the sanitizing treatment.

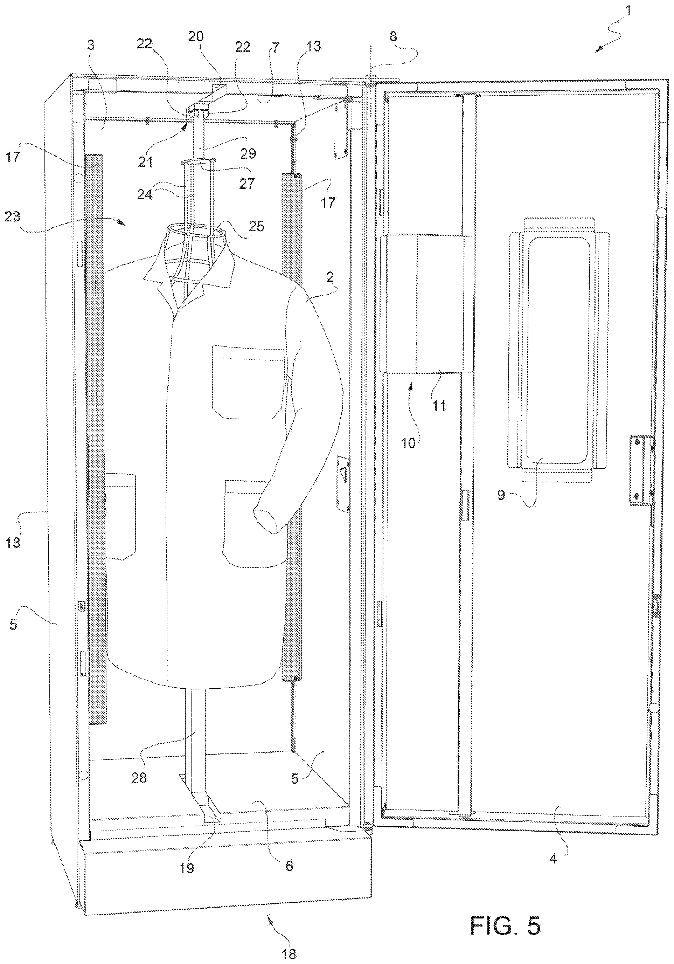

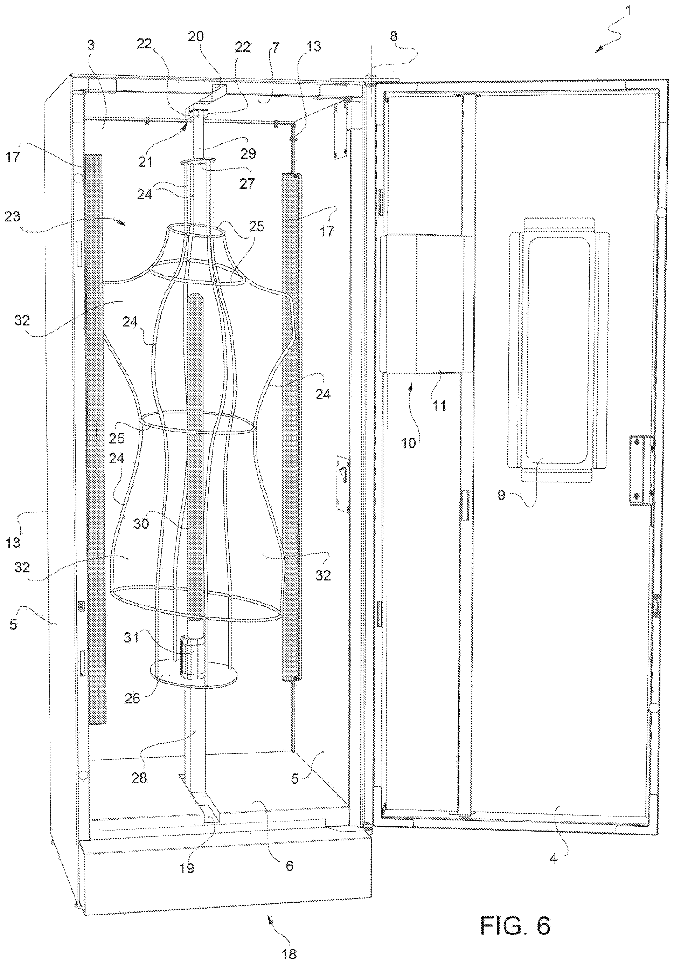

The variant shown in Figures 5 and 6 differs from the one shown in the preceding Figures solely in that the support device 14 is replaced with a support device 18 comprising a rectilinear lower guide 19 formed on the bottom wall 6, perpendicularly to the walls 3 and 4, and a rectilinear upper guide 20 formed on the upper wall 7, perpendicularly to the walls 3 and 4.

The device 18 further comprises a hook 21, which is in the form of a fork, comprises two elastically deformable arms 22, and protrudes downwards from the wall 7 at one end of the guide 20.

The device 18 further comprises a mannequin 23 comprising a plurality of shaped rods 24, which extend between the walls 6 and 7 and are mutually connected by means of a plurality of annular coupling elements 25 substantially parallel to the walls 6 and 7.

At least part of the rods 24 are fixed to a pair of end plates 26, 27 substantially parallel to the walls 6 and 7.

The plate 26 is arranged underneath the plate 27 and is provided with a lower coupling bar 28 slidably engaged in the guide 19, and the plate 27 is provided with an upper coupling bar 29 slidably engaged in the guide 20 and snap-fit between the arms 22 of the hook 21.

The mannequin 23 accommodates an Ultraviolet (UV) radiation source, which protrudes upwards from the plate 26 and is releasably coupled to a power connector 31 mounted on the plate 26.

The rods 24 and the elements 25 define therebetween a plurality of openings 32 to allow the UV radiations generated by the source 30 to sanitize an internal surface of the garment 2 arranged on the mannequin 23.

The rods 24 and the plates 26, 27 are oriented in such a manner that, when the mannequin 23 is mounted in the guides 19, 20 and locked in hook 21, the garment 2 on the mannequin 23 is arranged substantially parallel to the plane R.

In a variant not shown, the guide 20 and the bar 29 may be omitted and the mannequin 23 is provided with an upper coupling element configured to couple to the hook 15.

## Claims

1. A cabinet for sanitizing garments (2), comprising substantially parallel back and front walls (3, 4); a pair of substantially parallel side walls (5) connected to the back and front walls (3, 4) at respective longitudinal edges (13); and a top wall (7); **characterized by** further comprising a support device (14; 18) to maintain at least one garment (2) in a position substantially parallel to a reference plane (R) passing through two mutually opposite longitudinal edges (13); and two ultraviolet (UV) radiation sources (17) arranged on opposite sides of the reference plane (R).

2. The cabinet of claim 1, wherein the support device (14; 18) is coupled to the top wall (7).

3. The cabinet of claim 2, wherein the support device (14) comprises a hook (15) coupled to the top wall (7) and a cloth hanger (16) coupled to the hook (15).

4. The cabinet of claim 3, wherein the hook (15) extends in an arrangement plane (C) substantially perpendicular to the reference plane (R).

5. The cabinet of claim 3 or 4, wherein the hook (15) is L-shaped or U-shaped.

6. The cabinet of claim 2, wherein the support device (18) comprises a hollow mannequin (23) which accommodates a further ultraviolet (UV) radiation source (30) and comprises a plurality of openings (32) to allow the further UV radiation (30) to sanitize an internal surface of a garment (2) arranged on the mannequin (23).

7. The cabinet of claim 6, wherein the support device (18) comprises an attachment fork (21) protruding from the top wall (7), and an upper coupling bar (29) protruding from the mannequin (23) to snap-fit to the attachment fork (21).

8. The cabinet of claim 7, wherein the top wall (7) comprises an upper guide (20) slidably engaged by the upper coupling bar (29).

9. The cabinet of any one of claims 6 to 8, further comprising a bottom wall (6) comprising a lower guide (19); wherein the mannequin (23) comprises a lower coupling bar (28) slidably engaged in the lower guide (19).

10. The cabinet of any one of the preceding claims, further comprising an electronic control unit (10) comprising a memory (11) to store a plurality of sanitizing programmes, and a selection keypad (12) to select the sanitizing programmes.

11. The cabinet of any one of the preceding claims, wherein the front wall (4) is movable between an open position and a closed position and comprises a transparent inspection window (9).

12. The cabinet of any one of the preceding claims, wherein the ultraviolet (UV) radiation sources (17) are arranged in proximity of two longitudinal edges (13) on opposite sides of the reference plane (R).

## Patentansprüche

1. Ein Schrank zum Desinfizieren von Kleidungsstücken (2), der Folgendes aufweist:
im Wesentlichen parallele Rück- und Vorderwände (3, 4);
ein Paar im Wesentlichen paralleler Seitenwände (5), die mit den Rück- und Vorderwänden (3, 4) an jeweiligen Längskanten (13) verbunden sind; und
eine obere Wand (7);
**dadurch gekennzeichnet, dass** er ferner Folgendes aufweist:
eine Haltevorrichtung (14; 18), um mindestens ein Kleidungsstück (2) in einer Position zu halten, die im Wesentlichen parallel zu einer Referenzebene (R) ist, die durch zwei einander gegenüberliegende Längskanten (13) verläuft;
und zwei Ultraviolett-(UV)-Strahlungsquellen (17), die auf gegenüberliegenden Seiten der Referenzebene (R) angeordnet sind.

2. Der Schrank nach Anspruch 1, wobei die Haltevorrichtung (14; 18) mit der oberen Wand (7) gekoppelt ist.

3. Der Schrank nach Anspruch 2, wobei die Haltevorrichtung (14) einen an die obere Wand (7) gekoppelten Haken (15) und einen an den Haken (15) gekoppelten Kleiderbügel (16) aufweist.

4. Der Schrank nach Anspruch 3, wobei sich der Haken (15) in einer Anordnungsebene (C) erstreckt, die im Wesentlichen senkrecht zur Bezugsebene (R) steht.

5. Der Schrank nach Anspruch 3 oder 4, wobei der Haken (15) L- oder U-förmig ist.

6. Der Schrank nach Anspruch 2, wobei die Haltevorrichtung (18) eine Schaufensterpuppe (23) aufweist, die eine weitere Ultraviolett (UV)-Strahlungsquelle (30) aufnimmt und eine Vielzahl von Öffnungen (32) aufweist, damit die weitere UV-Strahlung (30) eine Innenfläche eines auf der Schaufensterpuppe (23) angeordneten Kleidungsstücks (2) desinfizieren kann.

7. Der Schrank nach Anspruch 6, wobei die Haltevorrichtung (18) eine von der oberen Wand (7) vorstehende Befestigungsgabel (21) und eine von der Schaufensterpuppe (23) vorstehende obere Koppelungsstange (29) zum Einrasten in die Befestigungsgabel (21) aufweist.

8. Der Schrank nach Anspruch 7, wobei die obere Wand (7) eine obere Führung (20) aufweist, in die die obere Koppelungsstange (29) gleitend eingreift.

9. Der Schrank nach einem der Ansprüche 6 bis 8, der ferner eine unteren Wand (6) aufweist, die eine untere Führung (19) aufweist; wobei die Schaufensterpuppe (23) eine untere Koppelungsstange (28) aufweist, die gleitend in die untere Führung (19) eingreift.

10. Der Schrank nach einem der vorangehenden Ansprüche, der ferner eine elektronischen Steuereinheit (10) aufweist, die einen Speicher (11) zum Speichern einer Vielzahl von Desinfektionsprogrammen und eine Auswahltastatur (12) zum Auswählen der Desinfektionsprogramme aufweist.

11. Der Schrank nach einem der vorhergehenden Ansprüche, wobei die Vorderwand (4) zwischen einer offenen und einer geschlossenen Stellung beweglich ist und ein transparentes Sichtfenster (9) aufweist.

12. Der Schrank nach einem der vorhergehenden Ansprüche, wobei die ultravioletten (UV) Strahlungsquellen (17) in der Nähe zweier Längskanten (13) auf gegenüberliegenden Seiten der Bezugsebene (R) angeordnet sind.

## Revendications

1. Armoire pour la désinfection de vêtements (2), comprenant des parois arrière et avant (3, 4) sensiblement parallèles ; une paire de parois latérales (5) sensiblement parallèles reliées aux parois arrière et avant (3, 4) au niveau de bords longitudinaux respectifs (13) ; et une paroi supérieure (7) ; **caractérisée en ce qu'**elle comprend en outre un dispositif de support (14 ; 18) pour maintenir au moins un vêtement (2) dans une position sensiblement parallèle à un plan de référence (R) passant à travers deux bords longitudinaux (13) mutuellement opposés ; et deux sources (17) de rayonnement ultraviolet (UV) disposées sur des côtés opposés du plan de référence (R).

2. Armoire selon la revendication 1, dans laquelle le dispositif de support (14 ; 18) est couplé à la paroi supérieure (7).

3. Armoire selon la revendication 2, dans laquelle le dispositif de support (14) comprend un crochet (15) couplé à la paroi supérieure (7) et un cintre (16) couplé au crochet (15).

4. Armoire selon la revendication 3, dans laquelle le crochet (15) s'étend dans un plan d'agencement (C) sensiblement perpendiculaire au plan de référence (R).

5. Armoire selon la revendication 3 ou 4, dans laquelle le crochet (15) est en forme de L ou en forme de U.

6. Armoire selon la revendication 2, dans laquelle le dispositif de support (18) comprend un mannequin creux (23) qui héberge une autre source (30) de rayonnement ultraviolet (UV) et comprend une pluralité d'ouvertures (32) pour permettre à l'autre rayonnement UV (30) de désinfecter une surface interne d'un vêtement (2) disposé sur le mannequin (23).

7. Armoire selon la revendication 6, dans laquelle le dispositif de support (18) comprend une fourche de fixation (21) faisant saillie depuis la paroi supérieure (7), et une barre de couplage supérieure (29) faisant saillie depuis le mannequin (23) pour s'encliqueter à la fourche de fixation (21).

8. Armoire selon la revendication 7, dans laquelle la paroi supérieure (7) comprend un guide supérieur (20) en prise de manière coulissante par la barre de couplage supérieure (29).

9. Armoire selon l'une quelconque des revendications 6 à 8, comprenant en outre une paroi inférieure (6) comprenant un guide inférieur (19) ; dans laquelle le mannequin (23) comprend une barre de couplage inférieure (28) en prise de manière coulissante dans le guide inférieur (19).

10. Armoire selon l'une quelconque des revendications précédentes, comprenant en outre une unité de commande électronique (10) comprenant une mémoire (11) pour stocker une pluralité de programmes de désinfection, et un clavier de sélection (12) pour sélectionner les programmes de désinfection.

11. Armoire selon l'une quelconque des revendications précédentes, dans laquelle la paroi avant (4) est mobile entre une position ouverte et une position fermée et comprend une fenêtre d'inspection transparente (9) .

12. Armoire selon l'une quelconque des revendications précédentes, dans laquelle les sources (17) de rayonnement ultraviolet (UV) sont disposées à proximité de deux bords longitudinaux (13) sur des côtés opposés du plan de référence (R).
